# EUROPEAN PATENT APPLICATION

(11) **EP 4 813 195 A1**
(43) Date of publication of application: **30.09.2026**
(21) Application number: 26168524.2
(22) Date of filing: 27.03.2026
(51) Int. Cl.: A01G 7/04, A01G 9/24, A61L 2/10

(54) **DEVICE FOR RADIATING A TARGET SURFACE SUCH AS A ROW OF PLANTS, AND ASSOCIATED METHOD**

(30) Priority: 27.03.2025 BE 202505180
(71) Applicant: Bogaerts Next Engineering BV, 2320 Hoogstraten (BE)
(72) Inventor: BOGAERTS, Joris Joannes, 2960 Brecht (BE)
(74) Representative: Oryon NV

(57) **Abstract**

The invention relates to a device for radiating a target surface, comprising a base provided with means for motion and a space-geometric arrangement of two or more parallel oblong-shaped UV-C lamps with length L provided in a plane or lamp frame that is parallel to the target surface, whereby the longitudinal axis of the lamps forms an angle α with the movement axis, and the distance D between two adjacent parallel lamps is equal to $D = \frac{L sin α}{n}$ whereby n is a factor for the compactness of the number of lamps in the plane. The invention also relates to a method for treating a target surface with such a device.

## Description

The present invention relates to a device for UV crop protection.

To combat pests and diseases of crops in greenhouses, horticulturalists use a range of techniques.

A commonly used method of crop protection is spraying. A chemical crop protection product is sprayed onto the desired areas, which may or may not be mixed with water. By finely spraying the mixture, hard-to-reach spots are also treated and the dosage is very uniform.

Depending on the crop, various treatment methods are possible, such as a central misting installation that covers the entire area. This involves a big installation cost. Smaller crops can sometimes be transported past a fixed treatment station.

For larger crops, a sprayer is typically driven along a row of plants. The dose can be controlled by aligning the spray rate with the driving speed.

Another method of crop protection is radiation. The plant is exposed for a certain period of time to a specific intensity of electromagnetic radiation (light). It is possible to apply the radiation in such a way that any diseases and pests are killed without causing significant damage to the crop. Crop protection through radiation is gaining popularity due to the often strict regulations regarding the use of chemical crop protection products.

The best-known form of radiation is UV-C treatment. This concerns ultraviolet light of category C, usually with a wavelength of 254 nm. Various UV-C lamps are available and are also used in other sectors for disinfection.

WO2021212199A1 describes a known UV treatment robot. The UV lamps are arranged in a plane parallel to the ground (0°) and radiate the crops from above.

It is very important for growers to be able to control the dosage accurately. Too low a dose has not effect. Too high a dose can endanger the health of people and animals. Moreover, chemical crop protection products are often strictly regulated.

UV-C treatment must also be applied in a controlled manner. Too low a dose has no effect, too high a dose damages the plant and the fruit.

US4387952A, US2018092308A1 and US2011037000A1 are examples of existing technologies for dosing radiation uniformly over a surface.

However, there is a problem regarding the efficient application of UV-C treatments to crops. Although UV-C lamps are available in different embodiments, there is a preference for a particular type. The reasons for this can be very diverse, such as cost, availability, fragility, complexity of the replacement procedure, ease of mounting, etc.

Consider an example in which oblong-shaped, narrow UV-C lamps with a length of 500 mm are used. The crop to be treated is arranged in long rows. The space between the rows is limited. The target surface of the crop has a height of 800 mm.

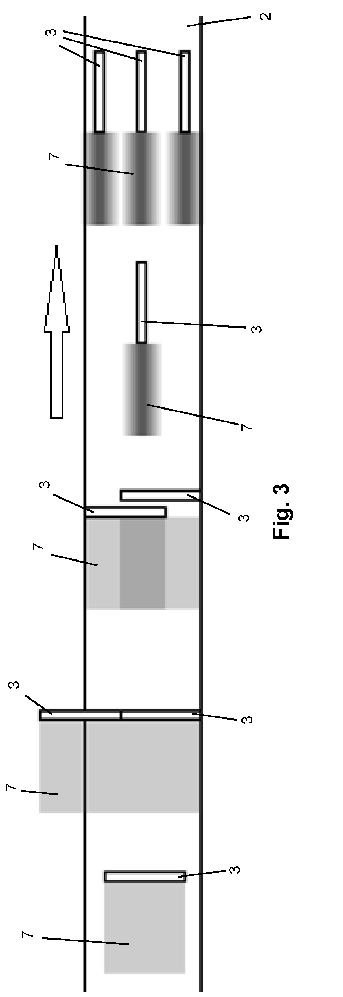

Figure 3 shows a vertical target surface, such as a row of plants, along which UV-C lamps mounted on a base provided with means for movement are moved in the direction of the arrow while the lamps radiate the target surface. The dark zones show the accumulated radiation distribution or the radiation trail of the lamps as a result of the motion. The figure shows five examples (a,b,c,d,e) of known space-geometric arrangements of UV-C lamps, each on the assumption that the lamps (via a robot or other drive mechanism) move along the crop (target area or target surface) in the direction indicated by the arrow. The target area is a crop of a certain height, with a high and low section (line under and above) and a central section. The movement direction is horizontal.

The target area may also be a horizontal plane of a certain width, such as the upper side of a target surface.

The radiation trail of the relevant arrangements relates to the part of the target area that is radiated by the lamp(s) during the horizontal movement. An efficient radiation requires a uniform radiation trail.

There is a problem when a non-uniform accumulated radiation distribution or a non-uniform radiation trail arises. This means that the radiation distributions of the individual lamps overlap, such that the target area receives more or less radiation locally, i.e. a non-uniform radiation distribution.

The terms radiation dose, (accumulated) radiation distribution, radiation trail, radiation area and target surface shall be understood to mean: target surface and radiation area are used interchangeably and refer to the surface on which a uniform dose of UV-C light is desired. The radiation distribution is the spatial distribution of the time-integrated irradiance on the target surface. Accumulated radiation distribution is the radiation distribution wherein the movement of the lamp is taken into account. To keep the distinction with the radiation distribution clear, we therefore refer to the accumulated radiation distribution as the radiation trail. The radiation dose is a term to indicate accumulated radiation distribution and is used in a more general context where the focus is on the result on the target surface.

A radiation area is optimally radiated by two or more lamps when the radiation areas of the individual lamps do not overlap and yet align optimally next to or against one another.

Some examples are explained here below.

Arrangement a relates to a single lamp arranged vertically and moved along the crop in the direction of the arrow, i.e. perpendicular to the length of the lamp. The central section of the target area receives a higher dose than the two outer sections. The radiation trail is not uniform.

Arrangement b relates to two lamps arranged vertically and moved along the crop in the direction of the arrow. The lamps are arranged in each other's extension. The joint length of the lamps amounts to 1000mm. Consequently, however, a considerable amount of radiation falls outside the target area of 800 mm, i.e. above the crop.

In arrangement c, two lamps are arranged vertically and moved along the crop in the direction of the arrow, wherein the lamps are arranged such that the first lamp is arranged 300mm higher than the second. Consequently, however, the central section of the target area receives twice as much radiation as the outer section.

Arrangement d relates to a single lamp arranged horizontally and moved along the crop in the direction of the arrow, i.e. lengthwise of the lamp. The central section of the target area therefore receives a much higher dose than the high and low sections.

In arrangement e, several lamps with a regular spacing are arranged horizontally and in parallel. However, the parts of the target area located directly behind a lamp, i.e. the radiation area of that lamp, receive a higher dose than the parts located between the lamps. Adding more lamps improves the result, but the non-uniformity remains, as overlapping radiation may then occur.

The problem of a non-uniform radiation trail arises particularly when the lamps are located close to the target surface, which is usually the case with the application of crop radiation.

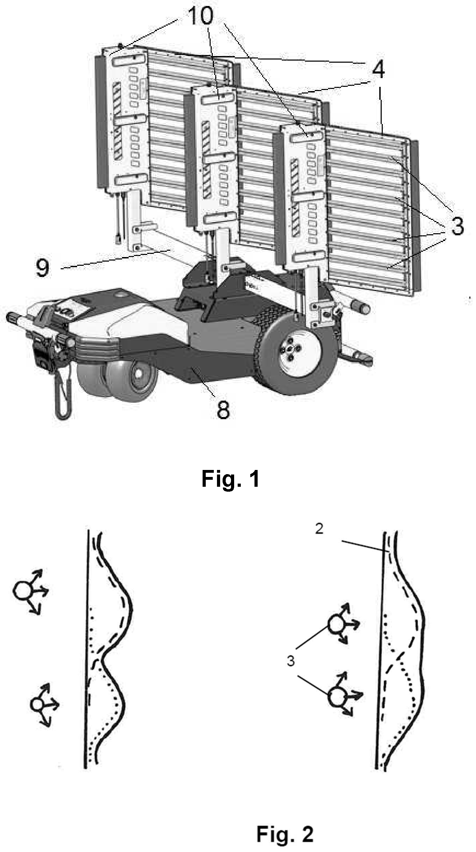

This is shown in figure 2 which shows a side view of the target surface radiated by two lamps (arranged lengthwise and horizontally). The curves indicate the spatial distribution of the irradiance. The closer the lamps are arranged to the target surface, the greater the ripple in the total irradiance. If there is more distance between the lamps and the target surface, the combined effect of the lamps barely shows any ripple.

Another condition is the orientation of the lamp. The problem of non-uniformity usually occurs where the target surface is located at a right angle under the lamp.

The purpose of the present invention is to provide a solution to at least one of the aforementioned and other disadvantages.

To this end, the invention relates to a device for radiating a target surface such as a row of plants, the device comprising a base provided with means for motion that is movable according to a movement axis parallel to the longitudinal axis of the target surface and a space-geometric arrangement of two or more parallel oblong-shaped UV-C lamps with length L provided in a plane or lamp frame that is parallel to the target surface and is provided on the aforementioned base, whereby the longitudinal axis of the lamps forms an angle α with the movement axis that is greater than 0° and less than 90° and the distance D between the first or second ends of two adjacent parallel lamps and measured along an axis perpendicular to the movement axis is equal to $D = \frac{L sin α}{n}$ whereby n is greater or equal to 1 and is a factor for the compactness of the number of lamps in the plane, and shows the number of incidences plus one with other active lamps measured on a fictitious axis parallel to the movement axis.

The factor n is a natural number greater than or equal to zero, whereby n shows the number the incidences plus one with other lamps located on a fictitious axis parallel to the movement axis between a first end of a first lamp and a second end of a second lamp.

The distance D is the regular spacing between the longitudinal axes of the parallel active elements or lamps, measured on an axis perpendicular to the movement axis.

The invention also relates to a space-geometric arrangement of active elements with a substantially oblong-shaped character for treating a target surface. The arrangement is such that the development of the active elements is uniformly distributed over the target surface. To this end the active elements are movable along the target surface.

Two or more active elements are arranged as follows, wherein the longitudinal axis of the active elements is parallel to the target surface and forms an angle with the movement axis, the longitudinal axis of the active elements is parallel to each other and the active elements are arranged such that they can be moved along a movement axis parallel to the longitudinal axis of the target surface.

In a specific embodiment of the present invention the active elements move along a vertical target surface. In another embodiment of the present invention, the active elements move along a horizontal target surface.

In a specific embodiment of the present invention, the device provides several active elements, for example two, which treat two parallel target surfaces simultaneously. The device moves the active elements centrally between the target surfaces. The distance between the active elements is provided to this end or dynamically adjustable by means of a distance control unit. The active elements have their effect on either side of a target surface or row of plants.

The invention also relates to a device that can move the active elements along the target surface. This is, for example, a robot on wheels or propelled in another way, for example on a rail provided between the target surfaces or rows of plants, or mounted at the bottom or at the top of the zone between the rows of plants.

The invention also relates to a controller which can control the activity of the active elements in combination with the speed of the device, to thus control the exposure of the target surface to the active elements.

The device is executed such that it can function autonomously. Indeed, UV-C radiation is dangerous for humans.

The device is executed such that it can also move in another direction than the movement axis, in order to successively move along different target surfaces.

In a specific embodiment, the active elements are germicidal lamps.

In a preferred embodiment, the active elements are identical.

Advantages of the device according to the invention are that the target surface receives a uniform dose of UV-C radiation. This is particularly beneficial where the lamps are located close to the target surface, which is usually the case with the application of crop radiation. Preferably, to this end the perpendicular distance between the UV-C lamps and the target surface is less than the spacing D between two adjacent lamps.

The geometry of the lamps in the plane is such that the radiation of the lamps is distributed uniformly over the target surface.

The target surface can be vertical or horizontal. In the case of crop radiation, the target surface is vertical and the movement is horizontal between the rows of plants. The movement of the arrangement between the rows of plants can be provided at the bottom of the rows, via the ground passages or at the top via a rail system or the like.

The characteristics of the device are further described in the claims.

The lamps in the lamp frame have a regular spacing D and preferably are identical. The height of the target surface, in case of a vertical target surface, partly determines the minimum number of active UV-C lamps.

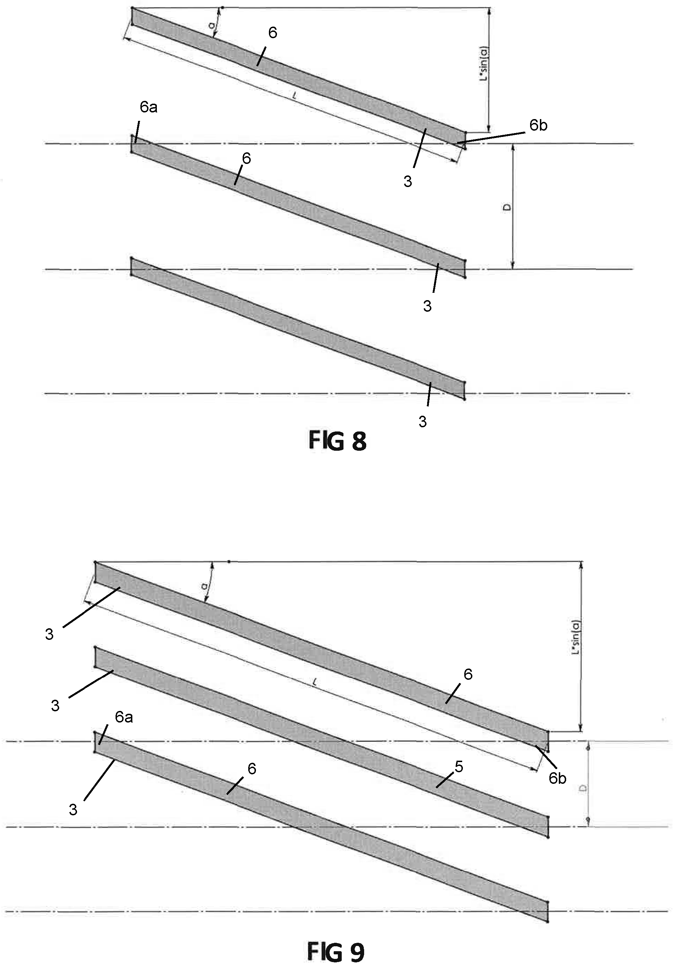

In a certain preferred embodiment of a device according to the invention, a desired spacing D between two adjacent lamps is the spacing according to figure 8, whereby the ends of two adjacent lamps, the first end of a first lamp and the second end of a second lamp, lie on a fictitious axis parallel to the movement axis without the axis intersecting another lamp. Consequently, there is no overlap between the radiation area or radiation trail of both lamps.

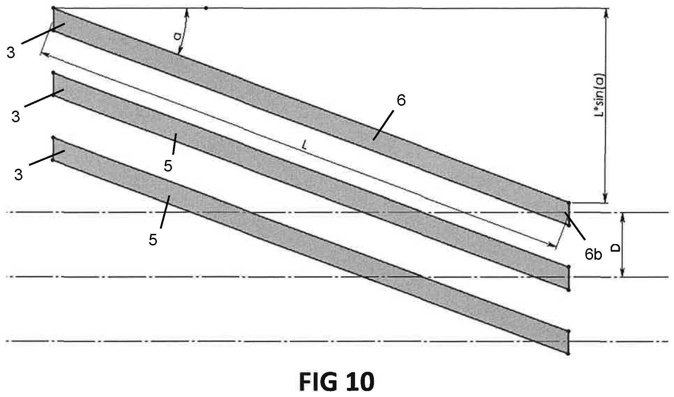

When the spacing D becomes too small, for example as shown in figure 9, whereby the axis intersects an additional lamp, too much overlap occurs in the radiation areas of the lamps. In figure 10, the spacing D is even less and therefore the overlap of radiation on the target surface is even greater. The crop thus receives extra radiation, which is usually unwanted.

However, said effect may indeed be desired. By setting the spacing D, the effect of overlap can be avoided or obtained. For example, it is known that gas discharge elements have a somewhat lower radiance at the ends than along the remainder of the length of the lamp.

The aforementioned arrangement may occur in an adjusted version wherein the spacing is reduced to take into account a reduced effectiveness at the ends of the lamps. The spacing ***D*** then becomes: $D = \frac{L sin α}{n} - ϵ$ whereby ε is a correction distance or offset to compensate for the reduced radiation intensity at the ends of the oblong-shaped active element. The offset ε is greater than zero and less than the distance D. When ε=0 there is no offset.

In a special preferred embodiment of a device according to the invention, the spacing D is reduced to take into account a reduced effectiveness at the ends of the lamps. The spacing D is then partly determined by a correction distance or offset ε which is determined by a zone of reduced radiation intensity at the ends of the oblong-shaped lamps. To determine the factor n, abstraction is made of, or no account is taken of, the offset and the incidence in the offset zone of the lamps.

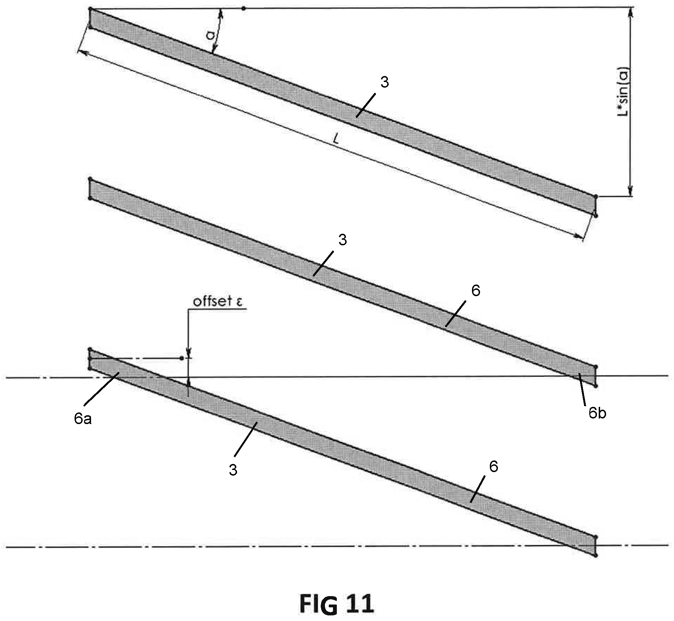

The correction distance or offset ε is shown in figure 11.

Preferably, the device comprises several of the aforementioned space geometric arrangements for simultaneously radiating several target surfaces during one and the same movement. Figure 1 shows such a device with three lamp frames which can therefore radiate four rows of plants simultaneously.

In a preferred embodiment of a device according to the invention, the arrangement is adjustable according to the desired radiation dose on the target surface for a greater or smaller overlap of radiation, to an optimal radiation without overlap but taking into account the reduced intensity of the ends of the lamps.

To this end the lamps of an arrangement of two or more UV-C lamps can be activated individually such that the distance D between two active UV-C lamps is adjustable.

Further to this end, the angle α of an arrangement of two or more UV-C lamps is adjustable according to the distance D between two adjacent active lamps.

Both adjustments, i.e. the angle and activation of lamps, can be made via a control module connected to the lamp frame. In this way, the device can be adapted quickly and easily to the rows of plants and the required radiation per crop or severity of pests and diseases.

Yet another advantage is that the radiation dose is simply adaptable by controlling the angle and/or the number of lamps and providing a greater overlap of radiation. Such effect cannot be obtained via a vertical or horizontal arrangement of the lamps.

The adjustment of the angle α, the activation of certain UV-C lamps as well as the distance D in the arrangement are determined depending on the desired radiation dose of the target surface.

In a specific embodiment of a device according to the invention, n = 1 whereby in a lamp frame there is no additional lamp between two adjacent lamps, the respective first and second ends of which are located on an axis parallel to the movement axis. In this case, the invention relates to a space-geometric arrangement with length L, angle α and spacing D whereby n = 1 provides an optimal radiation trail whereby the width/height of the lamp frame, measured between the extreme ends of the extreme lamps in the lamp frame, is at least as big is as the width/height of the target surface.

In another specific embodiment of a device according to the invention, n = 2 whereby in each lamp frame one additional lamp is located between two adjacent lamps, the respective first and second ends of which being located on an axis, resulting in a 100% overlap or double radiation dose to the target surface. In this case, the invention relates to a space-geometric arrangement with length L, angle α and spacing D wherein n = 2 provides an optimal radiation trail with 100% overlap or double irradiance wherein the width/height of the lamp frame measured between the extreme ends of the extreme lamps in the lamp frame is at least as big as the width/height of the target surface.

In a specific embodiment, the space-geometric arrangement comprises two or more parallel oblong-shaped active elements.

The present invention is also applicable to other active elements than UV-C lamps, such as for example oblong-shaped heat elements.

Another application consists of brushes that sweep across a surface, whereby each part of the surface must be touched by the same number of bristles.

In a variation of a device according to the invention, the lamps in the frame are not parallel but the longitudinal axes of two adjacent lamps form an angle equal to twice α. For example, one lamp is then arranged at an angle α to the movement axis and the adjacent lamp at an angle -α, whereby the lamps have an end that lies on the same axis parallel to the movement axis, such that the combined effect of all active elements accumulated over an axis parallel with the movement axis, is the same and provides a uniform radiation trail.

In this alternative embodiment, the longitudinal axis of one or more lamps forms an angle α with the movement axis and the longitudinal axis of one or more lamps forms an angle -α with the movement axis, whereby the angle -α is as big but opposite to the angle α. In this embodiment, the lamps are coplanar but not parallel. The distance D between the centres of the lamps centrally located on the longitudinal axis between the one end and the other end of a lamp, of two adjacent lamps and measured along an axis perpendicular to the movement axis complies with the formula for the distance D in this embodiment.

The invention also relates to a method for treating a target surface with a device according to the invention, the method comprising a. moving the device along the target surface according to the movement axis, whereby the UV-C lamps are parallel to the target surface and at a perpendicular distance from the aforementioned target surface, whereby the distance is less than the spacing D between two adjacent lamps, and b. uniformly radiating the target surface.

The method relates to the uniform radiation of the target surface which comprises a single (n = 1) or double (n = 2) radiation dose in the same step.

In a preferred embodiment of a method, the target surface is vertical and the movement axis is horizontal.

With the intention of better showing the characteristics of the invention, a preferred embodiment is described of a device according to the invention hereinafter, by way of an example without any limiting nature, with reference to the accompanying drawings wherein:
figure 1 schematically shows a preferred embodiment of a device according to the invention;
figure 2 shows the spatial distribution of irradiance of two lamps on a surface;
figure 3 shows (a-e) five space-geometric arrangements of UV-C lamps;
figure 4 shows a preferred embodiment of an arrangement of UV-C lamps (n = 1);
figure 5 shows an arrangement of UV-C lamps (n ≈ 1.5) whereby the target surface is not uniformly treated;
figure 6 shows an arrangement of UV-C lamps (n = 2) with an overlap in UV-C radiation;
figure 7 shows a preferred embodiment of an arrangement of UV-C lamps with an offset whereby the partial overlap compensates for the reduced effectiveness at the ends;
figure 8 shows an arrangement of UV-C lamps as shown in figure 4 whereby the radiation distributions of the lamps connect and do not overlap (n = 1);
figure 9 shows an arrangement of UV-C lamps as shown in figure 6 whereby the radiation distributions of the lamps overlap 100% (n = 2, one additional lamp);
figure 10 shows an arrangement of UV-C lamps whereby the radiation distributions of the lamps overlap 200% (n = 3, two additional lamps);
figure 11 shows a preferred embodiment of an arrangement of UV-C lamps (as shown in figure 7a) with an offset whereby the partial overlap compensates for the reduced effectiveness at the ends;
figure 12 shows a number of variants of a lamp frame with on the left 10 lamps and n=2, and on the right 6 lamps and n=1.

Figure 1 schematically shows a preferred embodiment of a device 1 according to the invention. The device relates to a UV-C crop protection robot 8 provided with three parallel lamp frames 4.

The device is a UV-C crop protection robot 8 on wheels which is movable at a constant speed according to a movement axis parallel to the longitudinal axis of the target surface, whereby the robot is provided with three parallel lamp frames 4.

The device is built as a mobile robot provided with a mobile base on wheels. The robot is movable at a constant speed according to a movement axis parallel to the longitudinal axis of the target surface. The robot drives between the rows of plants from row to row.

A support frame is mounted on the base that serves as primary structure for attaching modular functional components comprising one or more lamp frames and the respective control unit per lamp frame.

Three lamp frames are provided at a spacing from each other in this example. The design is therefore scalable to be able to cover different treatment widths.

The frames 4 are slideable and mounted parallel to each other on a horizontal axis 9. The distance between the frames is adjustable according to the distance between the rows of plants. The distance between a frame and a row of plants determines an efficient radiation and depends on the crop type.

Each frame 4 contains, in the plane of the frame, eleven oblong-shaped UV-C lamps 3 arranged in parallel with a length at an angle of approximately 20°. Each frame is also connected to a control module 10 which optionally can set or control the angle and activation per lamp.

For figure 2 and 3 we refer to the explanation in the relevant paragraphs above.

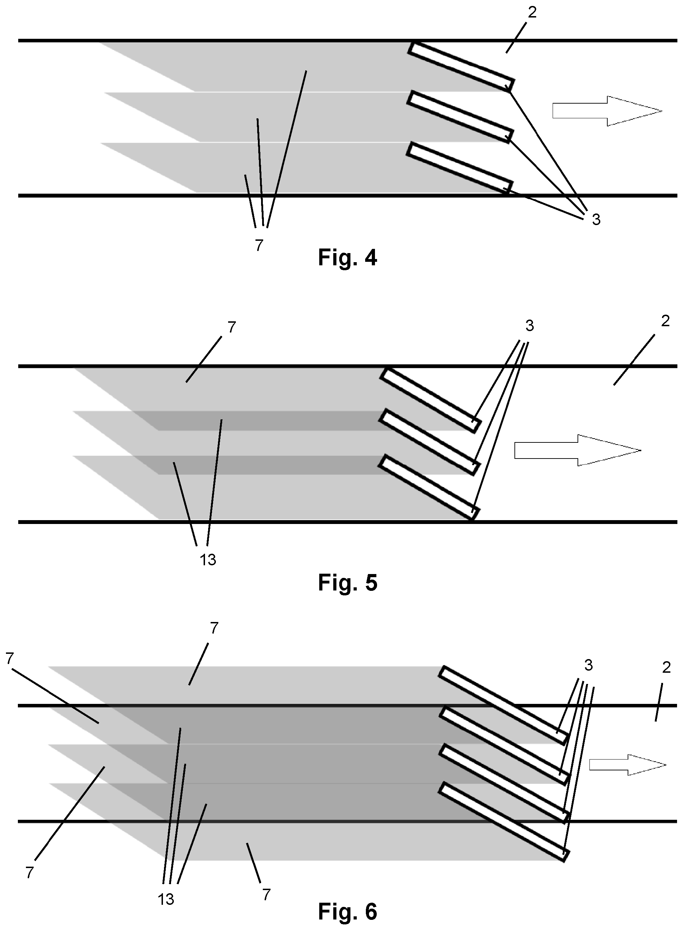

Figure 4 is a preferred embodiment of an arrangement of UV-C lamps with n = 1. Each lamp 3 has a radiation trail 7 that does not overlap with the radiation trail of another lamp. The distance D is optimal, with the exception of a reduced intensity and a reduced radiation trail, at the ends of the lamps.

Figure 5 shows an arrangement of UV-C lamps (n ≈ 1.5) whereby the target surface 2 is not uniformly treated. The central section 11 of the lamps 3 treats the target surface once, the extreme sections 12 of the lamps provide an overlap 13 such that the target surface receives extra radiation.

Figure 6 shows an arrangement of UV-C lamps 3 (n = 2) with another 100% overlap in radiation trail 7. The target surface 2 is radiated twice.

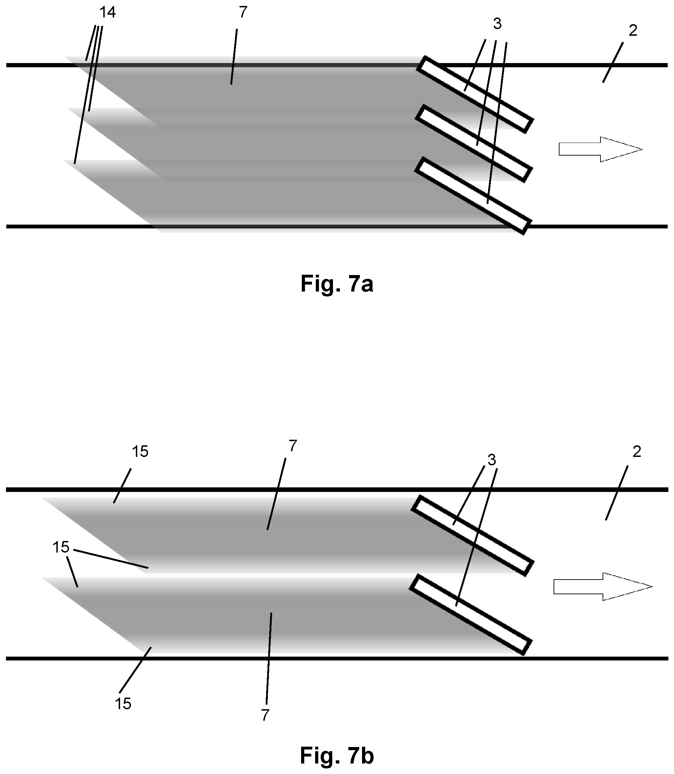

Figure 7 is a preferred embodiment of an arrangement of three UV-C lamps 3 with an offset whereby the partial overlap at the ends of the lamps compensates for the reduced effectiveness at said ends. As a result, the radiation of the target surface 2 is optimal.

By way of example, the arrangement with two UV-C lamps 3 shows the reduced effectiveness at the ends more clearly.

Figure 8 shows an arrangement of UV-C lamps 3 as shown in figure 4 whereby the radiation trails of the lamps connect and do not overlap (n = 1).

Figure 9 shows an arrangement of UV-C lamps 3 as shown in figure 6 whereby the radiation trails of the lamps overlap 100% (n = 2). One additional lamp 5 is provided between every two UV-C lamps 6 for a 100% overlap or double radiation of the target surface.

Figure 10 shows an arrangement of UV-C lamps 3 whereby the radiation trails of the lamps overlap 200% (n = 3). Two additional lamps 5 are provided between every two UV-C lamps 6 for a 200% overlap.

Figure 11 shows a preferred embodiment of an arrangement of UV-C lamps 3 for an optimal radiation (as shown in figure 7a), with an offset wherein the partial overlap compensates for the reduced effectiveness at the ends. In this example n=1.

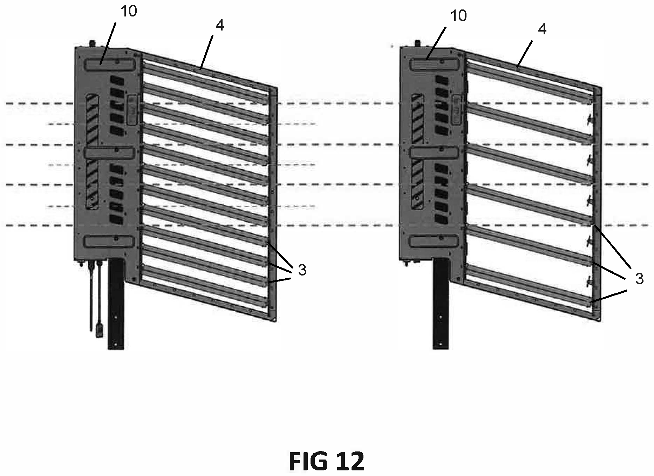

Figure 12 shows a number of variants of a lamp frame with on the left ten lamps and n=2, and on the right six lamps and n=1. The left variant has overlapping radiation trails 7, the right variant does not. In the right variant, an intermediate lamp is deactivated or not mounted.

This principle can be expanded. For example, in an arrangement with n=3, the accumulated radiation distribution is uniform if, successively, two lamps are alternated with one lamp deactivated/omitted, but also if two lamps are deactivated and one lamp activated. Numerous variations can be conceived in this way.

The present invention is by no means limited to the embodiments described as an example and shown in the drawings, but a device according to the invention as defined by the claims can be realised according to all kinds of variants without departing from the scope of the invention.

## Claims

1. Device (1) for radiating a target surface (2) such as a row of plants, the device comprising a base provided with means for motion that is movable according to a movement axis (arrow) parallel to the longitudinal axis of the target surface and a space-geometric arrangement of two or more parallel oblong-shaped UV-C lamps (3) with length L provided in a plane or lamp frame (4) that is parallel to the target surface (2) and is provided on the aforementioned base, **characterised in that** the longitudinal axis of the lamps (3) forms an angle α with the movement axis that is greater than 0° and less than 90° and the distance D between the first or second ends (6a, 6b) of two adjacent parallel lamps (3) and measured along an axis perpendicular to the movement axis is equal to $D = \frac{L sin α}{n}$ whereby n is greater or equal to 1, and is a factor for the compactness of the number of lamps (3) in the plane (4), and shows the number of incidences plus one with other active lamps (6) measured on a fictitious axis parallel to the movement axis.

2. Device according to claim 1, **characterised in that** the perpendicular distance between the UV-C lamps (3) and the target surface (2) is less than the spacing D.

3. Device according to claim 1 or 2, **characterised in that** the geometry of the lamps (3) in the plane (4) is such that the radiation (7) of the lamps is uniformly distributed over the target surface.

4. Device according to any one of the previous claims, **characterised in that** the target surface (2) is vertical and the movement axis horizontal.

5. Device according to any one of the previous claims, **characterised in that** the lamps (3) in the plane (4) have a regular spacing D.

6. Device according to any one of the previous claims, **characterised in that** the two or more lamps (3) are identical.

7. Device according to any one of the previous claims, **characterised in that** the distance D is reduced by a correction distance or offset ε determined by the reduced radiation intensity at the ends of the oblong-shaped lamps, whereby D is equal to $D = \frac{L sin α}{n} - ϵ$ and ε is greater than zero and less than D.

8. Device according to any one of the previous claims, **characterised in that** the device comprises several aforementioned space-geometric arrangements (3, 4) for simultaneously radiating several target surfaces (2) during one and the same movement.

9. Device according to any one of the previous claims, **characterised in that** the device is a UV-C crop protection robot (8) which is movable at a constant speed according to a movement axis parallel to the longitudinal axis of the target surface, whereby the robot is provided with three parallel lamp frames (4).

10. Device according to any one of the previous claims, **characterised in that** the arrangement (3, 4) is adjustable according to the desired radiation dose on the target surface (2) for a greater or smaller overlap of radiation distribution.

11. Device according to any one of the previous claims, **characterised in that** the lamps (3) of an arrangement of two or more UV-C lamps (3) can be individually activated such that the distance D between two active UV-C lamps (3) is adjustable.

12. Device according to any one of the previous claims, **characterised in that** the setting of the angle α and the activation of certain UV-C lamps (3) and the distance D in the arrangement are determined depending on the desired radiation dose on the target surface (2).

13. Method for treating a target surface with a device according to any one of the claims 1-12, the method comprising a. moving the device along the target surface according to the movement axis, whereby the UV-C lamps are parallel to the target surface and at a perpendicular distance from the aforementioned target surface, whereby the distance is less than the spacing D between two adjacent lamps, and b. uniformly radiating the target surface.

14. Method according to claim 13, **characterised in that** the uniform radiation of the target surface comprises a single (n = 1) or double (n = 2) radiation intensity in the same step.

15. Method according to claim 13 or 14, **characterised in that** the target surface is vertical and the movement axis is horizontal.
